# EUROPEAN PATENT APPLICATION

(11) **EP 2 348 433 A2**
(43) Date of publication of application: **27.07.2011**
(21) Application number: 11250065.7
(22) Date of filing: 21.01.2011
(51) Int. Cl.: G06F 19/00

(54) **Diabetes management unit, method and system**

(30) Priority: 22.01.2010 US 297566 P
(71) Applicant: Lifescan, Inc., Milpitas, CA 95035 (US)
(72) Inventor: Ray, Pinaki, Fremont, CA 94539 (US); Matian, Greg, Foster City, CA 94404 (US); Harmon, Kirk, San Ramon, CA 94583 (US)
(74) Representative: Tunstall, Christopher Stephen

(57) **Abstract**

A system and method are shown and described for outputting a customized health data report with minimal usage of a user interface.

## Description

This application claims the benefits under 35 USC §119 and 120 of prior provisional application S.N. 61/297566 on January 22, 2010, which is hereby incorporated by reference into this application in its entirety.

### BACKGROUND

Diagnostic medical devices that test patient body fluids to provide a snapshot of a particular disease state hold a lot of valuable data. The value of this data can be harnessed most effectively by software that downloads the data and presents intelligent analytical reports that can be used by healthcare professionals to make quick and informed therapy decisions.

Data management software is not widely used by healthcare professional (HCP) offices due to the time needed and the skill level of the staff needed to install and run software on a PC. The value of the reports generated by the software is appreciated by most healthcare professionals but the time spent and cost involved in getting familiar with new software created by multiple vendors, invoking it, navigating thru screens downloading device data and then generating and printing reports is prohibitive. Most data management software requires users to do additional data mining (date range, name of patient, report type, etc.) before a report is presented to them. Some vendors have created dedicated hardware solutions (for example, hardware that prints reports when connected to a blood glucose meter). This has its own drawbacks from the standpoint of taking up precious space in an HCP office, requiring additional ink and paper for printing, lack of the ability to customize reports, lack of the ability to change download behavior (i.e., add a patient name, require name authentication), lack of the ability to store data for historical trending, additional expenditure on a dedicated hardware etc. Also, the staff will need to be trained to operate the hardware. In some cases, these dedicated printers are provided in the front office for use by patients. This often creates issues with the Health Insurance Portability and Accountability Act of 1996 (HIPAA) since private health data is visible to other patients.

In addition, most desktop software and dedicated printers do not provide any audiovisual alerts related to patient compliance when downloading data from meters.

### Summary of the Disclosure

In one embodiment, a method of transferring data from a medical device to a data management unit is provided. The data management unit is configured to communicate with a plurality of medical device types. The method may be achieved by: generating with the microprocessor, different customized reports specific to different medical device types; coupling the at least one type of the plurality of medical device types to the data management unit; identifying with the microprocessor, that the at least one type of medical device types has been coupled to the data management unit; providing an input into the user interface to trigger the output of customized report specific to the at least one type of medical device; and outputting the customized report.

In yet another embodiment, a diabetes management system is provided that includes a data management unit, at least one medical device. The data management unit is configured to generate different customized reports specific to different medical device types based on a medical device type being coupled to the data management unit; and the data management unit is configured to couple to the medical device so that the data management unit automatically identifies the medical device type, transfers data from the identified medical device, and outputs the customized report specific to the identified medical device after an input on a user interface.

In a further embodiment, a data management unit is provided that includes a microprocessor and a communication medium. The microprocessor receives data relating to diabetes from one or more medical device types. The communication medium is coupled to the microprocessor, the communication medium providing comparative data of a mean blood glucose, number of hyperglycemic incidence, number of day with test less than a specified value, and mean total daily dose of insulin collected over different periods of reporting.

These and other embodiments, features and advantages will become apparent to those skilled in the art when taken with reference to the following more detailed description of various exemplary embodiments of the invention in conjunction with the accompanying drawings that are first briefly described.

### Brief Description of the Figures

The accompanying drawings, which are incorporated herein and constitute part of this specification, illustrate presently preferred embodiments of the invention, and, together with the general description given above and the detailed description given below, serve to explain features of the invention (wherein like numerals represent like elements).

Figure 1 is a schematic illustration of a networked system with which data transferring and reporting software may be employed.

Figure 2 is a schematic illustration of an exemplary application of the subject software employed in a healthcare professional's office in which wireless communication is used;

Figure 3 is a schematic illustration of the a data transferring and reporting software;

Figure 4 is a schematic illustration of an Internet-based or local area network (LAN)-based system or environment with which the a data transferring and reporting software may be employed;

Figures 5A and 5B provide a flow chart of the process steps according to the methods of the data transferring and reporting software.

Figure 6 illustrates an exemplary graph illustrating glycemic event or trend information which can be provided by implementation of the data transferring and reporting software.

Figure 7 illustrates a diabetes management system that includes an analyte measurement and management device and data communication devices, according to an exemplary embodiment described and illustrated herein.

Figure 8A illustrates a top portion of a circuit board of the analyte measurement and management device, according to an exemplary embodiment described and illustrated herein.

Figure 8B illustrates a bottom portion of the circuit board of the analyte measurement and management device, according to an exemplary embodiment described and illustrated herein.

Figure 9 illustrates a schematic of the functional components of a therapeutic dosing device, according to an exemplary embodiment described and illustrated herein.

Figure 10 is a flow chart illustrating a method for transferring data from a medical device to a data management unit, according to an exemplary embodiment described and illustrated herein.

Figure 11 is a screen-shot of a user interface that allows a user to pre-select customized reports that correspond to a plurality of medical devices where such reports are outputted when there is a user input into the user interface, according to an exemplary embodiment described and illustrated herein.

Figure 12 is a screen-shot of a user interface that allows a user to pre-select another type of customized reports that correspond to a plurality of medical devices where such reports are automatically outputted when the type of medical device is recognized by the diabetes management unit and without user input into the user interface, according to an exemplary embodiment described and illustrated herein.

Figure 13 is a screen-shot of a user interface that instructs a user to couple a medical device to a diabetes management unit and prompts the user to input into the user interface to trigger the output of the pre-selected customized report, according to an exemplary embodiment described and illustrated herein.

Figure 14A is a screen-shot of a portion of a 14-Day Summary Report that includes a logbook, according to an exemplary embodiment described and illustrated herein.

Figure 14B is a screen-shot of another portion of a 14-Day Summary Report that includes a glucose trend graph, according to an exemplary embodiment described and illustrated herein.

Figure 15 is a screen-shot of a user interface for doing a population analysis, according to an exemplary embodiment described and illustrated herein.

Figure 16 is a screen-shot of a glucose trend graph that includes pre-meal, post-meal flags, and not flagged glucose measurements, according to an exemplary embodiment described and illustrated herein.

Figure 17 is a screen-shot of the glucose trend graph, in accordance with Figure 16, where the data is filtered for pre-meal flags, according to an exemplary embodiment described and illustrated herein.

### Detailed Description of the Exemplary Figures

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. In addition, as used herein, the terms "patient," "host," "user," and "subject" refer to any human or animal subject and are not intended to limit the systems or methods to human use, although use of the subject invention in a human patient represents a preferred embodiment.

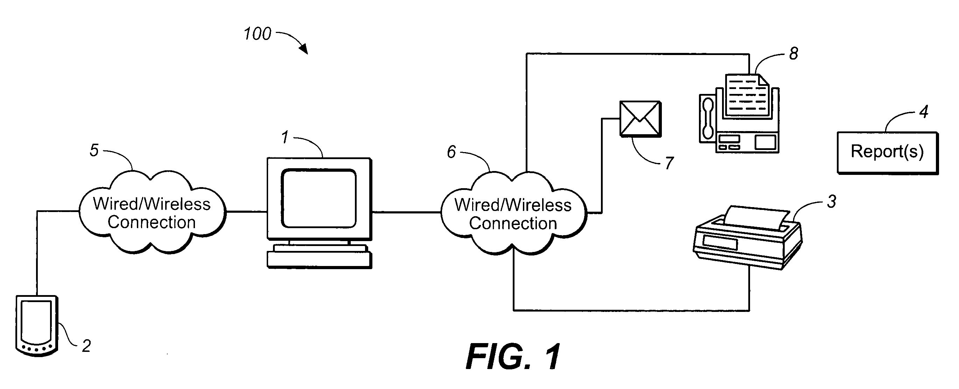

Figure 1 is a schematic illustration of a networked system 100 with which data transferring and reporting software may be employed. The subject software is hosted on a device 1, such as a PC or PDA. Device 1 is in hardwire or wireless communication with one or more medical device types 2, such as a blood glucose monitor, and one or more information outputs, such as a printer 3, email system 7 and facsimile machine 8. Medical device 2 is automatically detected by the host device 1 upon which data, e. g., patient-specific glucose event and trend information, medication compliance, etc., is automatically transferred from the diagnostic device 2 to the host device 1 using a wired/wireless connection 5 where the data is stored in a database or other memory for application, analysis and later acquisition. During the data transfer and storage in the database, visual and audio alerts are provided to the healthcare professional indicating patient compliance to established therapy rules, specific to disease type and patient medical history. Host device 1 then compiles, analyzes and organizes the data according to user-selected parameters. The information is then formatted into one or more reports 4 which are then sent automatically to one or more data outputs via wired/wireless connection 6. The data output may include but is not limited to a printer 3, a fax machine 8 and an electronic file which may be transmitted through email or the like.

Reports 4 can be generated in the form of text, graphs, matrix charts, pie charts, etc. Standard information that may be provided on a report includes but is not limited to patient name and account number, meter serial numbers, HCP office visitation log, etc. Also, the software may be configured to provide trend graphs that display causes of glycemic events, e.g., food, medication, exercise, stress, etc, in a unique iconic format. Additional color-coded alerts can be provided on the printed report 4 to assist the healthcare professional to assess data outside normal parameters and limits

The wired/wireless connection 5 and 6 may take the form of a wire, direct serial or USB cable connection; a TCP/IP or Ethernet based network connection; or a wireless connection using protocols like 802.11, infrared (IR) or radiofrequency (RF), e.g., Bluetooth. Detection of the diagnostic medical device 2 by host device 1 may be done automatically whereby medical device 2 identifies itself to host device 1 via an interrupt mechanism that notifies the host 1 that a device 2 wants to communicate with it. Alternatively, the host device 1 could continuously poll for any device 2 and initiate communication with it upon detection. Host device 2 may be configured, i.e., programmed accordingly, to automatically download data from device 2 and to print reports 4 upon establishing communication between the two devices by a hard connection, such as a cable or wired network, or by a wireless connection, such as by IR or RF signals, where the user has transmitted a signal prompting host device 1 of the desire to transfer records.

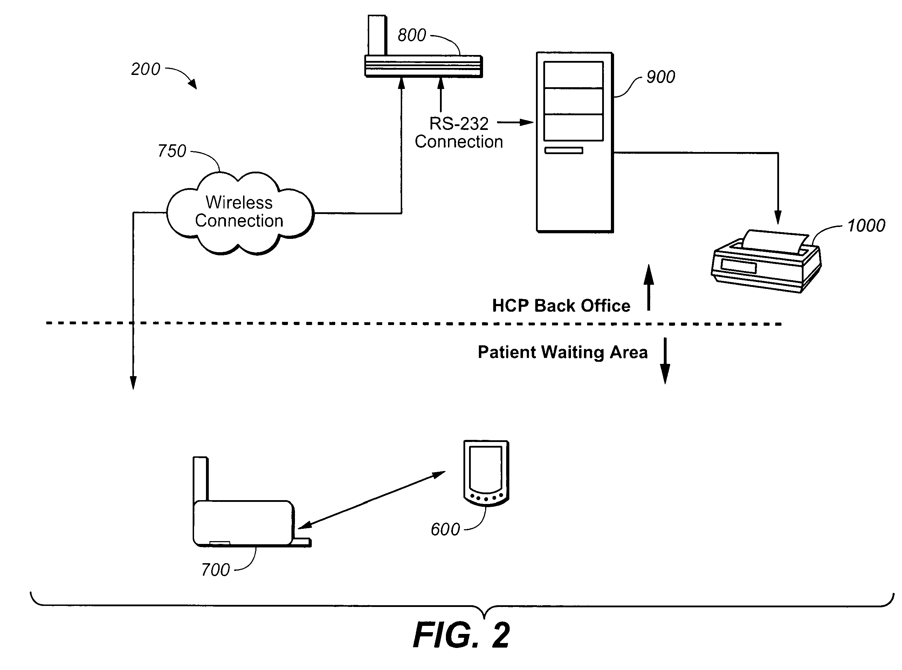

Figure 2 is a schematic illustration of an exemplary application 200 of the subject software program employed in an HCP office having a back office and a patient waiting area. A host device 900, such as PC or handheld device, e.g., PDA, cell phone, etc., loaded with the subject software service and program and a printer 1000, by which reports can be printed automatically for use by the healthcare personnel, are placed in the back office where patients would not have access to HIPAA regulated data. Wireless communication 750 is established between a medical device 600 brought in by a patient visiting the clinic and host system 900 by way of transceivers 700 and 800, the former of which is stationed in the patient waiting area and the latter of which is stationed in the back office. Transceiver 800 may be a stand-alone component connected to the communication port of the host 900 by way of a standard interface cable through an RS-232 compatible serial port, or may be integrated within host 900. Transceiver 700 may also be a standalone unit provided with a similar cable, which is connectable to medical device 600, or may be integrated into medical 600.

In an embodiment, host 900 periodically transmits a "FIND METER" command on to wireless transceiver 700 via transceiver 800. Wireless transceiver 700 in turn relays the FIND METER command to device 600. In response, device 600 transmits a device serial number back to host device 900, which recognizes the device serial number and associates it with a pre-existing patient code or establishes a new account for the patient. Alternatively, upon detection of a new meter/patient, the program can be configured to prompt the HCP office user to enter pertinent user data to add the new patient to the system. Host device 900 in turn transmits a signal back to device 600 requesting data transfer. The transferred data is then stored in host device 900 in association with the existing patient or newly established patient account (or keep it in temporary memory in another embodiment). A report module of the subject software runs the reports according to a standing order or an otherwise real-time request and sends them to printer 1000 automatically.

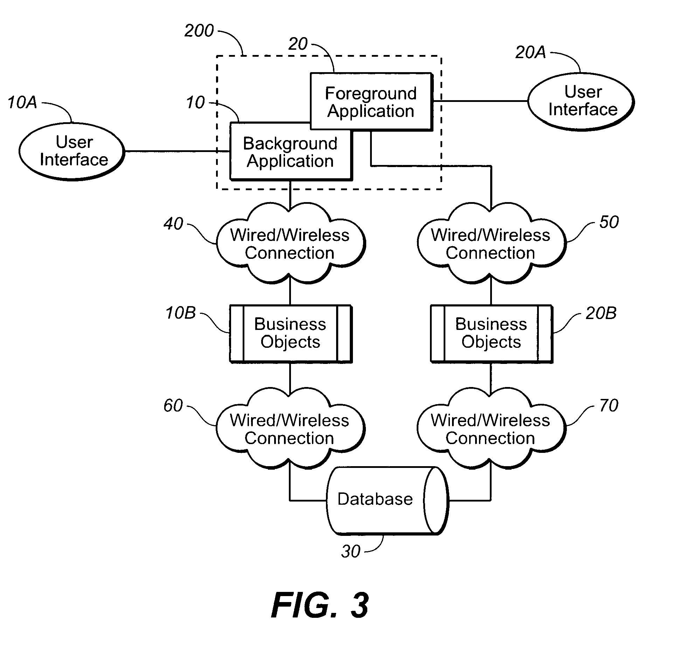

Figure 3 is a schematic illustration of the various application and/or data components or aspects of the data transferring and reporting software. A microprocessor can be programmed to generally carry out the steps of the data transferring and reporting software. The microprocessor can be part of a particular device, such as, for example, a glucose meter, an insulin pen, an insulin pump, a server, a mobile phone, personal computer, or mobile hand held device. The software program 200 may include a background or service application 10 and a foreground application 20. Background application runs continuously upon start up of a user's system. By way of user interface 10A, a healthcare professional can configure or customize the parameters of the background service 10 based on the rules established within the healthcare practice. Such rules may include but are not limited to the types of reports, the format the reports (e.g., print color) and the data included in the reports, etc. Subsequent to initial configuration of background application 10, further user interface is not necessary unless the rules need to be modified. Background application 10 accesses business objects 10B and other data stored in database 30 via wired/wireless connections 40 and 60, respectively. Foreground application 20 is only activated by user input. It has its own user interface 20A, by which a user may actively submit data or query for data output, and its own business objects 20B. Foreground application 20 accesses business objects 20B and other data stored in database 30 via wired/wireless connections 50 and 70, respectively. While the two applications may each have their own database, sharing database resources has the advantages of allowing the background application to access historical data (i.e., previously downloaded data) as well as real-time data (i.e., last download), and to access rules of the foreground application. The business objects 10A and 20A process patient-specific data against established rules which define goals/targets, alert thresholds, etc. which are stored in database 30. In particular, the business objects dictate the type and extent of data or information provide on a report produced by the system.

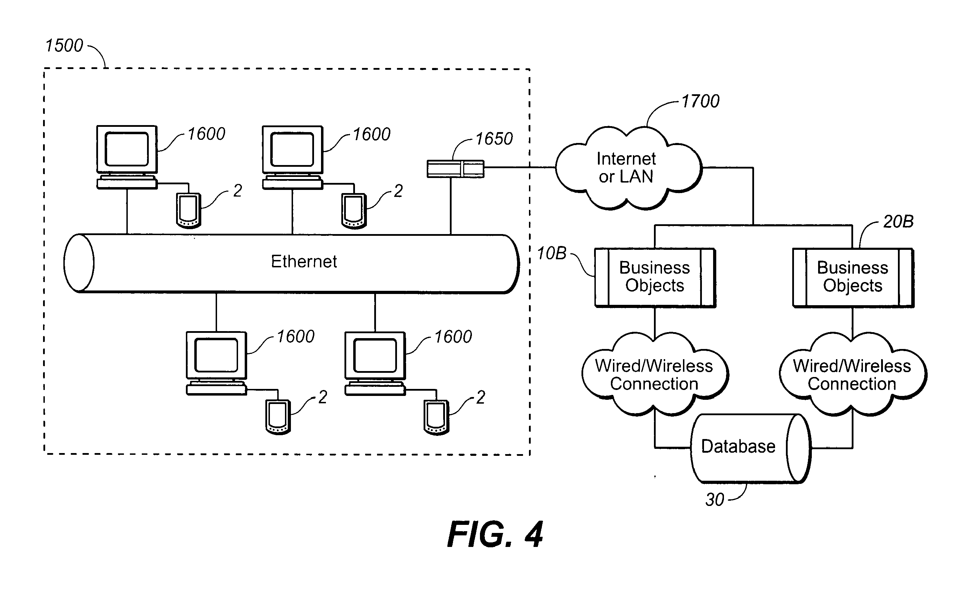

Figure 4 is a schematic illustration of a system network or environment with which the software and/or data components of Figure 3 may be employed. In such an environment, network 1500 may involve a network of multiple clients 1600 and/or individually serviced clients interconnected through the Ethernet and then routed through a router 1650. The client host device or system may be a PC or handheld devices, such as a PDA, which hosts background service 10 and foreground application 20 described above. A diagnostic device 2 and a printer 1000, as described above, may be connected to each of the client host systems 1600. The user interface for these clients may be browser based or Windows based. The network 1500 and individual clients 1600 may communicate with the services of the data transferring and reporting software via an Internet-based or local area network (LAN)-based.

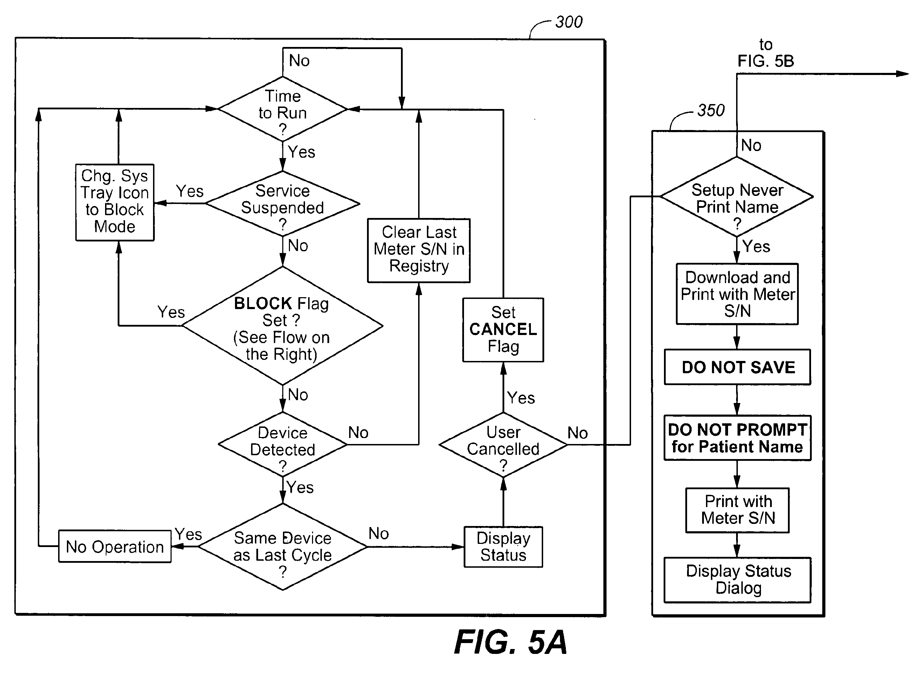

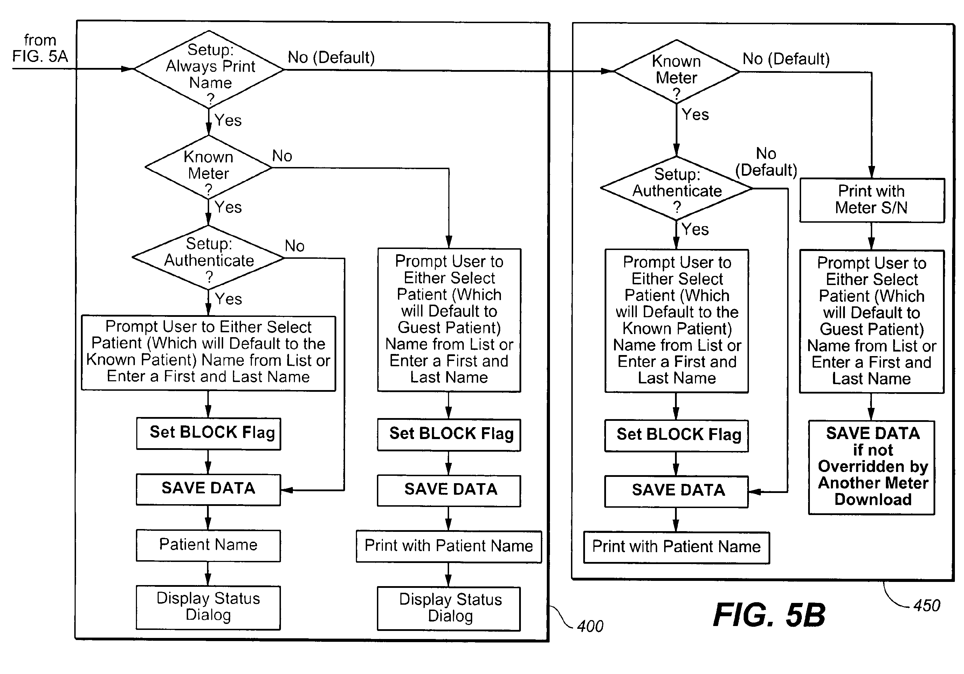

Figure 5 illustrates a flow chart of the operation of the system of the data transferring and reporting software. A microprocessor can be programmed to generally carry out the steps of the data transferring and reporting software. The microprocessor can be part of a particular device, such as, for example, a glucose meter, an insulin pen, an insulin pump, a server, a mobile phone, personal computer, or mobile hand held device. The main flow path 300 illustrates the basic handshaking that takes place between the background and foreground applications, discussed above with respect to Figure 3. The background application of the system operates in cycles referred to as scan cycles, whereby the service periodically wakes up from a sleep or wait mode and conducts a number of checks and executes certain instructions, after which the background service returns to a sleep or wait mode for a fixed period of time. When the background application is awake or active, the system scans for and is able to detect the presence of a designated medical device. If it is not suspended (due to activity in the foreground application), the service will check if it has been blocked by a pending action from the user (e.g. an open dialog box prompting the user to enter a patient name). If a designated device is detected, the background service determines whether the detected device is the same device that was last detected. If the same device is detected, the operation is ceased thereby preventing repetitive printing of the same report. However, if a different device is detected, the user is alerted to the fact by audio-visual cues, and the relevant data is automatically downloaded to the user's system from the device and printed, emailed or faxed according to a preconfigured report processing and printing scheme.

Before providing a detailed description of the report processing and printing configurations, the handshaking between the background and foreground applications is described with reference to main flow path 300. As the background and foreground applications share common functionality and resources when communicating with a medical device, only one application can be active at a time. As such, a mechanism may be required whereby each of the two applications can notify the other when the required resources are in use by it.

If a user attempts to perform any meter communication function from the foreground application when the background application is actively communicating with a meter, a message is sent to the user interface of the foreground application indicating that the background service is busy and that the user should try again later. Visa-versa, when the foreground application is actively communicating with a meter, the background application becomes suspended. Examples of actions or processes initiated by a user with the foreground application which will suspend the background application include but are not limited to displaying meter communication instruction pages; and activating the meter download, setup or clear screen (at least until the user exits the screen). Upon completion of the operation or transmission, the suspension is cleared. Meanwhile, the background service continuously checks the status of the flag when it is available to scan for a device.

Referring again to subroutines 350, 400 and 450, the system can be configured to store data and print reports in a customized manner. Typically, customization involves the amount of patient data that is to be printed on a report. For privacy and other administrative reasons, a HCP may wish to limit the amount of patient data that is provided in report and stored on its system. Subroutine 350 provides a report with minimal patient data and does not save any of the data in the user's system. In particular, the patient name is never used but instead, the service is configured to identify the patient according to the serial number of the detected medical device. On the other hand, the user may want the report to identify the patient by name, such as provided by each of subroutines 400 and 450. When the user (HCP) chooses to always have the patient name printed on reports, subroutine 400 executes. With subroutine 400, when the meter detected is unknown, the user is prompted to enter or select the patient name. This sets the BLOCK flag, putting the background service in a wait mode. The background service is not able to process/detect additional medical devices in this state. When a patient name is entered, the downloaded data, including the meter's serial number, is saved and associated with the patient name in a database and the BLOCK flag is removed. When the detected meter is "known" by the system (i.e., is one that corresponds to a previously existing patient account stored in the software database), prior to saving the data, the foreground application first authenticates or confirms whether the meter is still associated with previously identified patient or is now used by a different patient. This step ensures that every meter's data is associated with the correct patient in the database. When the user (HCP) chooses to have a report printed without any obstruction to the office workflow whatsoever, i.e., with minimum software prompts, subroutine 450 (preferably the default routine) is executed. That is, if the meter is known, a report is printed with the patient name associated with the meter, or if the meter is unknown to the software, a report is printed only with the meter's serial number. In the latter case, the user is then prompted to enter the patient name, and the background application is blocked. If this prompt goes unanswered, and another meter is detected at this time, the background application is unblocked.

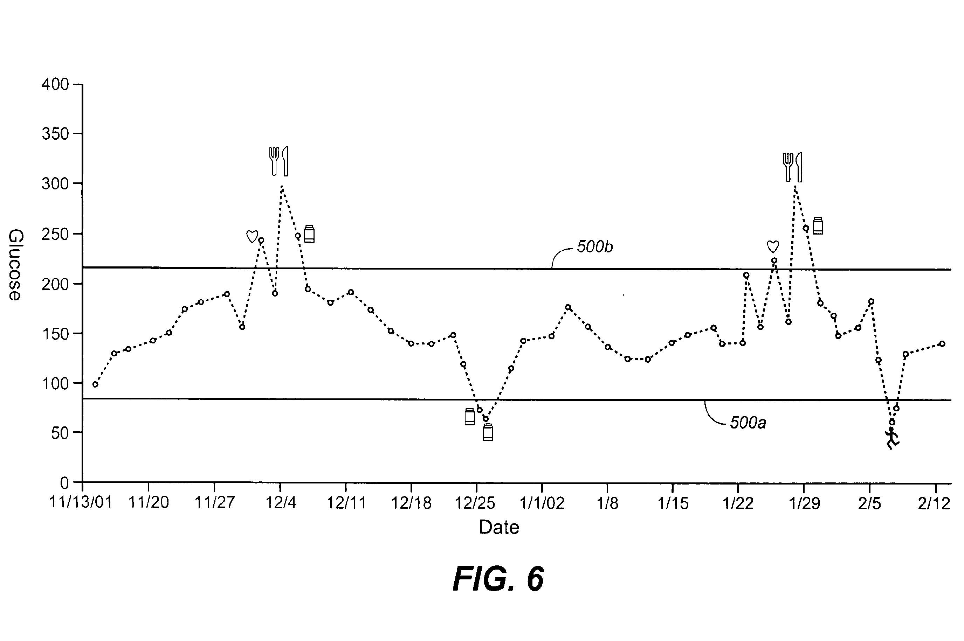

Figure 6 illustrates an example of one type of report that may be generated by the data transferring and reporting software. This report may be a glucose trend graph wherein the data points signify the occurrence of a glycemic event. Acceptable glucose levels fall between minimum and maximum values 500a and 500b. Data points or glycemic events falling below minimum value 500a and above maximum value 500b are tagged with various icons symbolic of the glycemic event (e.g., a heart signifying a stressful event, a fork and knife signifying that the patient has eaten; a pill bottle signifying that the patient has taken medication or insulin, and a running figure signifying that the patient has exercised). The glycemic events can be automatically tagged by the meter or can be manually entered by the user. An HCP can consider the collective events and the corresponding glucose trend in order to more effectively make recommendations to the patient regarding, e.g., food intake, exercise and medication administration. The system can be further configured whereby additional details, e.g., the specific type of event, date and time of the event or glucose measurement, the value of measurement, user comments, etc., of about an event may be displayed when a mouse is hovered over the corresponding event icon.

Also provided by the subject invention are kits for use in practicing the subject methods. The kits include software programs recorded on a CD-ROM, DVD or USB plug or the like, which programs may be downloaded to the meter, PDA and/or an external data device for use with the systems. Finally, the kits may further include instructions for customizing, calibrating and/or configuring subject devices and systems. These instructions may be present on one or more of the packaging, label inserts or containers within the kits, or may be provided on a CD-ROM or the like.

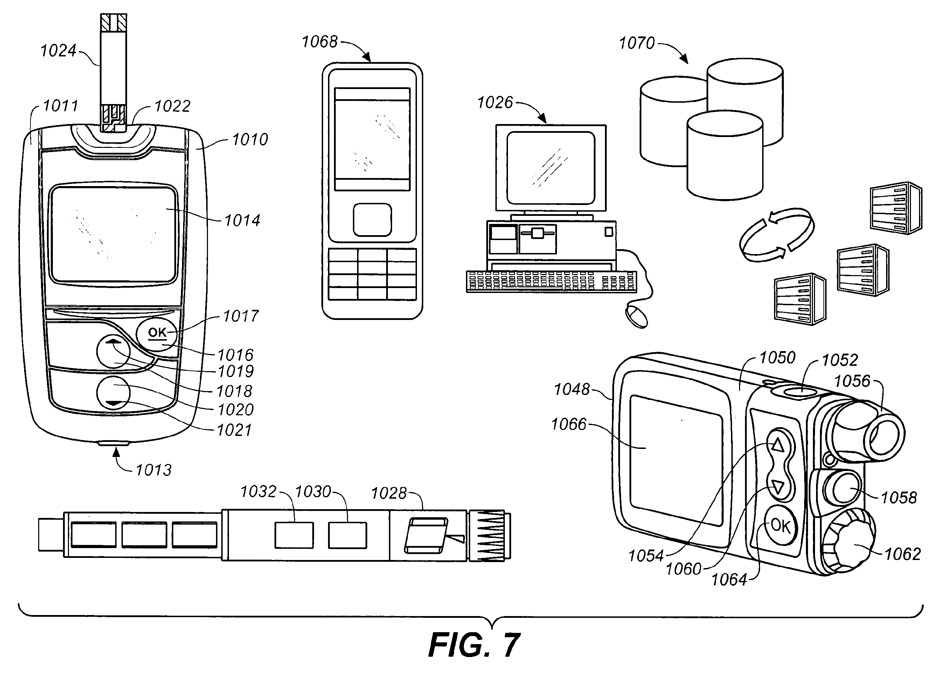

Figure 7 illustrates another embodiment of a diabetes management system that may include one or more medical device types and one or more data management units. Examples of a medical device may include a glucose meter 1010, an insulin pen 1028, an insulin pump 1048, and a continuous glucose monitor (not shown). Examples of a data management unit may include a mobile phone 1068, a personal computer 1026, and a server 1070. The medical devices can be configured to communicate with a data management unit either wirelessly or through a wired cable. Further, the medical device can be configured to communicate with a data management unit through a combination medical devices and/or data management unit. In addition, a medical device can be in the form of a combination with one or more of the above-mentioned medical devices such as, for example, an integrated device that can measure glucose and dispense insulin. In certain instances, a data management unit can also be integrated with the functionality of a medical device such as, for example, a glucose meter configured to process and output reports after receiving data from an insulin pump. In an embodiment, the data transferring and reporting software may be implemented using a personal computer where data is transferred from one or more medical device types such as for example, glucose meter 1010 and insulin pump 1048.

Note that recommendations, warnings, compliance updates, and reports may be annunciated to a user. As used herein, the term "user" is intended to indicate primarily a mammalian subject (e.g., a person) who has diabetes but which term may also include a caretaker or a healthcare provider who is operating the meter 10 on behalf of the diabetes subject. As used here, the term "annunciated" and variations on the root term indicate that an announcement may be provided via text, audio, visual or a combination of all modes of communication to a user, a caretaker of the user, or a healthcare provider.

Glucose meter 1010 can include a housing 1011, user interface buttons (1016, 1018, and 1020), a display 1014, a strip port connector 1022, and a data port 1013, as illustrated in Figure 7. User interface buttons (1016, 1018, and 1020) can be configured to allow the entry of data, navigation of menus, and execution of commands. Data can include values representative of analyte concentration, and/or information, which are related to the everyday lifestyle of an individual. Information, which is related to the everyday lifestyle, can include food intake, medication use, occurrence of health checkups, and general health condition and exercise levels of an individual. Specifically, user interface buttons (1016, 1018, and 1020) include a first user interface button 1016, a second user interface button 1018, and a third user interface button 1020. User interface buttons (1016, 1018, and 1020) include a first marking 1017, a second marking 1019, and a third marking 1021, respectively, which allow a user to navigate through the user interface.

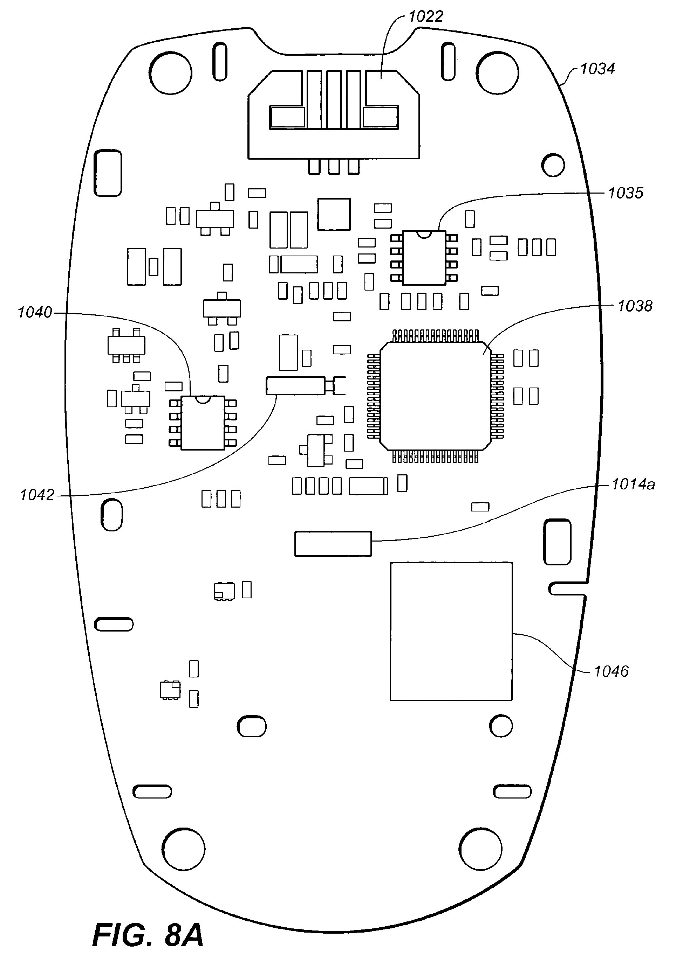

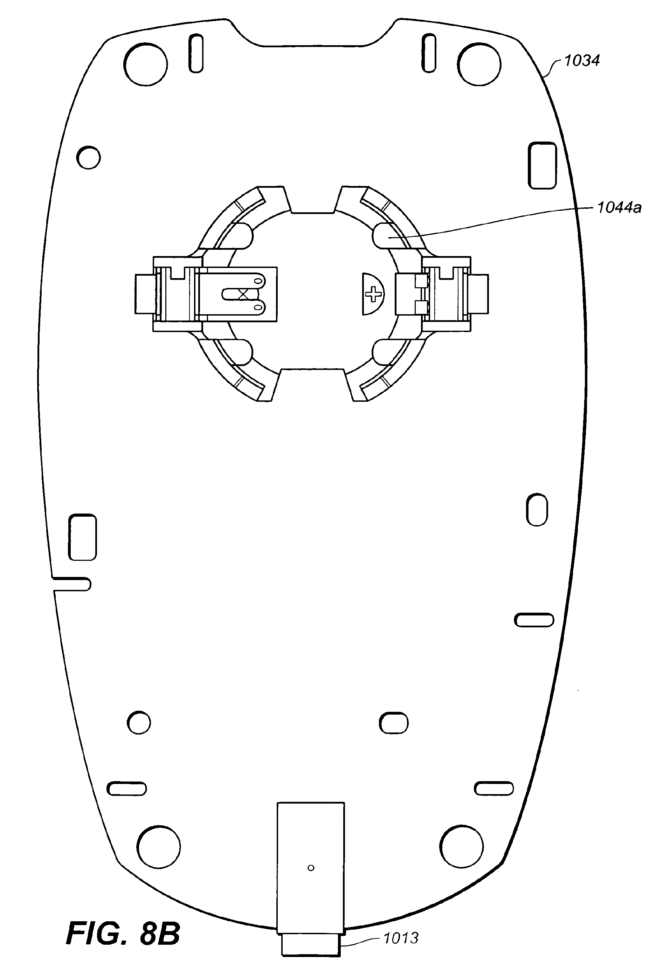

The electronic components of meter 1010 can be disposed on a circuit board 1034 that is within housing 1011. Figures 8A and 8B illustrate the electronic components disposed on a top surface and a bottom surface of circuit board 1034, respectively. On the top surface, the electronic components include a strip port connector 1022, an operational amplifier circuit 1035, a microcontroller 1038, a display connector 1014a, a non-volatile memory 1040, a clock 1042, and a first wireless module 1046. On the bottom surface, the electronic components include a battery connector 1044a and a data port 1013. Microcontroller 1038 can be electrically connected to strip port connector 1022, operational amplifier circuit 1035, first wireless module 1046, display 1014, non-volatile memory 1040, clock 1042, battery connector 1044a, data port 1013, and user interface buttons (1016, 1018, and 1020).

Referring to Figure 8A, operational amplifier circuit 1035 can include two or more operational amplifiers configured to provide a portion of the potentiostat function and the current measurement function. The potentiostat function can refer to the application of a test voltage between at least two electrodes of a test strip. The current function can refer to the measurement of a test current resulting from the applied test voltage. The current measurement may be performed with a current-to-voltage converter. Microcontroller 1038 can be in the form of a mixed signal microprocessor (MSP) such as, for example, the Texas Instrument MSP 430. The MSP 430 can be configured to also perform a portion of the potentiostat function and the current measurement function. In addition, the MSP 430 can also include volatile and non-volatile memory. In another embodiment, many of the electronic components can be integrated with the microcontroller in the form of an application specific integrated circuit (ASIC).

Strip port connector 1022 can be configured to form an electrical connection to the test strip. Display connector 1014a can be configured to attach to display 1014. Display 1014 can be in the form of a liquid crystal display for reporting measured glucose levels, and for facilitating entry of lifestyle related information. Display 1014 can optionally include a backlight. Data port 1013 can accept a suitable connector attached to a connecting lead, thereby allowing glucose meter 1010 to be linked to an external device such as a personal computer. Data port 1013 can be any port that allows for transmission of data such as, for example, a serial, USB, or a parallel port. Clock 1042 can be configured for measuring time and be in the form of an oscillating crystal. Battery connector 1044a can be configured to be electrically connected to a power supply.

In one exemplary embodiment, test strip 1024 can be in the form of an electrochemical glucose test strip, as illustrated in Figure 7. Test strip 1024 can include one or more working electrodes and a counter electrode. Test strip 1024 can also include a plurality of electrical contact pads, where each electrode can be in electrical communication with at least one electrical contact pad. Strip port connector 1022 can be configured to electrically interface to the electrical contact pads and form electrical communication with the electrodes. Test strip 1024 can include a reagent layer that is disposed over at least one electrode. The reagent layer can include an enzyme and a mediator. Exemplary enzymes suitable for use in the reagent layer include glucose oxidase, glucose dehydrogenase (with pyrroloquinoline quinone co-factor, "PQQ"), and glucose dehydrogenase (with flavin adenine dinucleotide co-factor, "FAD"). An exemplary mediator suitable for use in the reagent layer includes ferricyanide, which in this case is in the oxidized form. The reagent layer can be configured to physically transform glucose into an enzymatic by-product and in the process generate an amount of reduced mediator (e.g., ferrocyanide) that is proportional to the glucose concentration. The working electrode can then assay a concentration of the reduced mediator in the form of a current. In turn, glucose meter 1010 can convert the current magnitude into a glucose concentration.

Referring back to Figure 7, insulin pen 1028 can include a housing, preferably elongated and of sufficient size to be handled by a human hand comfortably. The device 1028 can be provided with an electronic module 1030 to record dosage amounts delivered by the user. The device 1028 may include a second wireless module 1032 disposed in the housing that, automatically without prompting from a user, transmits a signal to first wireless module 1046 of glucose meter 1010. The wireless signal can include, in an exemplary embodiment, data to (a) type of therapeutic agent delivered; (b) amount of therapeutic agent delivered to the user; or (c) time and date of therapeutic agent delivery.

In one embodiment, a therapeutic delivery device can be in the form of a "user-activated" therapeutic delivery device, which requires a manual interaction between the device and a user (for example, by a user pushing a button on the device) to initiate a single therapeutic agent delivery event and that in the absence of such manual interaction deliver no therapeutic agent to the user. A non-limiting example of such a user-activated therapeutic agent delivery device is described in co-pending U.S. Non-Provisional Application No. 12/407173 (tentatively identified by Attorney Docket No. LFS-5180USNP); 12/417875 (tentatively identified by Attorney Docket No. LFS-5183USNP); and 12/540217 (tentatively identified by Attorney Docket No. DDI-5176USNP), which is hereby incorporated in whole by reference. Another non-limiting example of such a user-activated therapeutic agent delivery device is an insulin pen 1028. Insulin pens can be loaded with a vial or cartridge of insulin, and can be attached to a disposable needle. Portions of the insulin pen can be reusable, or the insulin pen can be completely disposable. Insulin pens are commercially available from companies such as Novo Nordisk, Aventis, and Eli Lilly, and can be used with a variety of insulin, such as Novolog, Humalog, Levemir, and Lantus.

Referring to Figure 7, the therapeutic dosing device can also be a pump 1048 that includes a housing 1050, a backlight button 1052, an up button 1054, a cartridge cap 1056, a bolus button 1058, a down button 1060, a battery cap 1062, an OK button 1064, and a display 1066. Pump 1048 can be configured to dispense medication such as, for example, insulin for regulating glucose levels.

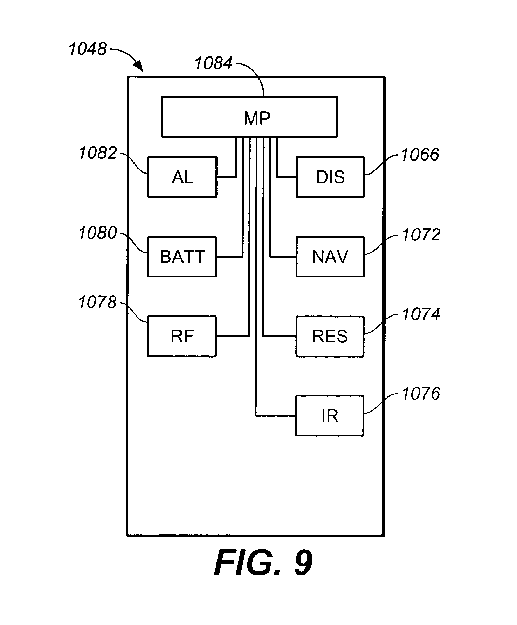

Referring to Figure 9, pump 1048 includes the following functional components that are a display (DIS) 1066, navigational buttons (NAV) 1072, a reservoir (RES) 1074, an infrared communication port (IR) 1076, a radio frequency module (RF) 1078, a battery (BAT) 1080, an alarm module (AL) 1082, and a microprocessor (MP) 1084. Note that navigational buttons 1072 can include up button 1054, down button 1060, and ok button 1064.

People with diabetes will often have to perform several glucose tests and administer several insulin doses to effectively manage the disease. The glucose concentration amount, time that the test was performed, and lifestyle data (e.g., meals and exercise) are factors often taken into account when calculating a proper insulin-dosing regimen. A user is often confronted with a large amount of data from more than one medical device that needs to be assimilated, comprehended, and processed into actionable next steps in managing the disease. Applicant believes that there is a need for a better way to aggregate and process the data into one data management unit to output specific reports for a particular medical device type that a user or health care professional (HCP) can easily understand. Applicant also believes that the user or HCP will want a first type of customized report for a particular medical device type periodically upon a requested input into the data management unit and a second type of customized report for a particular medical device type to be automatically outputted when the particular medical device is identified by the data management unit. The following will describe a data transferring and reporting software system that can provide a first customized report type upon a user input and also a second customized report automatically.

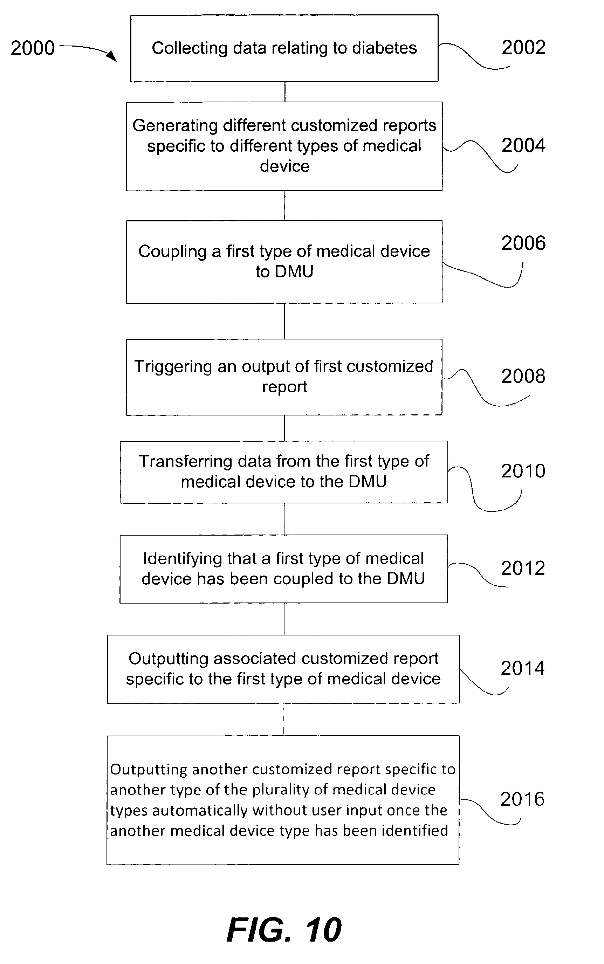

Figure 10 is a flow chart illustrating a method 2000 for transferring data from a medical device to a data management unit. A microprocessor can be programmed to generally carry out the steps of method 2000. The microprocessor can be part of a particular device, such as, for example, a glucose meter, an insulin pen, an insulin pump, a server, a mobile phone, personal computer, or mobile hand held device. Data relating to diabetes may be collected such as, for example, by assaying a plurality of glucose concentrations with a first type of medical device, as shown in a step 2002. The assaying may include inserting an electrochemical test strip 24 into strip port connector 22 of the first type of medical device (e.g., glucose meter 10). The user interface may then prompt a user to dose blood onto test strip 24. Glucose meter 10 can then perform the assay and output a glucose result on display 14. After the result is calculated, the glucose result and time stamp can be saved to a memory in the glucose meter 10. The term time stamp can refer to the date and time that an event was performed, which in this case is a glucose measurement.

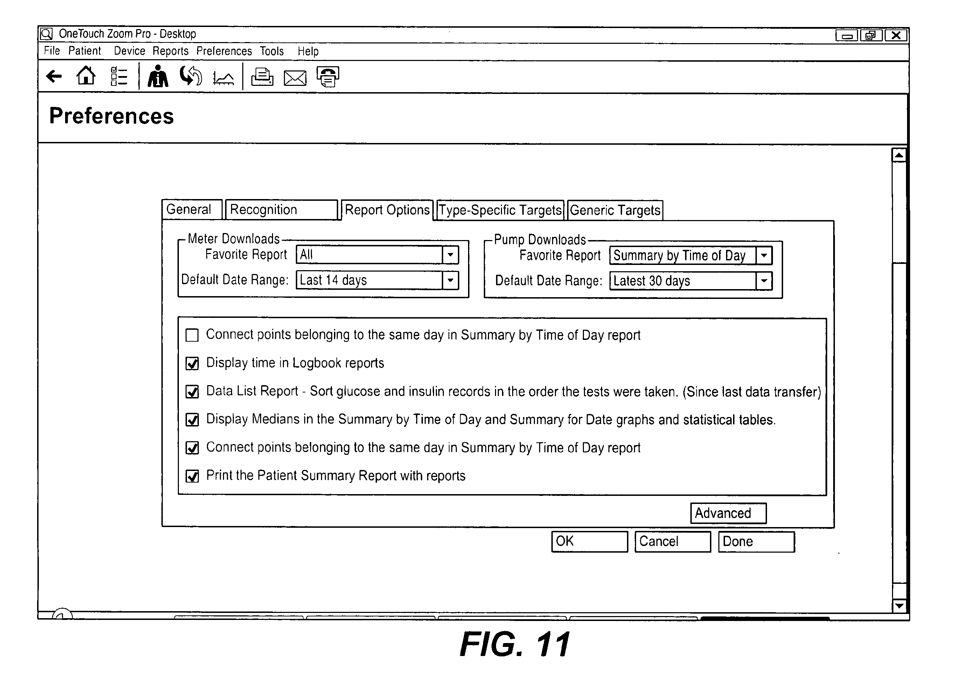

As illustrated in a step 2004 of Figure 10, a user can pre-select or generate at the time or beforehand one or more customized reports, where each report corresponds to a particular type of medical device, when there is an input into a user interface. Figure 11 illustrates an exemplary screen-shot of a user interface that allows a user to pre-select two types of customized reports that correspond to a glucose meter and an insulin pump. Such customized reports can be annunciated when a particular medical device is coupled to the data management unit and there is an input into a user interface.

Examples of reports may be Summary by Time of Day, Summary by Date, Summary by Day of Week, Detailed Logbook, Data List, Summary Logbook, Glucose Distribution, and 14-Day Summary. The following will describe the exemplary reports in more detail.

The Summary by Time of Day report may include messages triggered by the Pattern Recognition analysis, a scatter plot of all glucose data over a 24-hour time axis, and a scatter plot of insulin data over a 24-hour time axis. An example of Pattern Recognition analysis can be found in U.S. Pre-Grant Publication No. 2008/0234992, which is hereby fully incorporated by reference herein with a copy provided in the Appendix.

The Summary by Date report may include messages triggered by the Pattern Recognition analysis, a scatter plot of all glucose data over the report date range, and a bar graph of total daily insulin over the report date range.

The Summary by Day of Week report may include messages triggered by the Pattern Recognition analysis, a scatter plot of all glucose data for each day of the week, and a scatter plot of insulin data for each day of the week.

The Detailed Logbook report may include messages triggered by the Pattern Recognition analysis, glucose, insulin, and carbohydrate data for each of the defined time slots.

The Data List report may include all result types and values from one or more medical device types such as total daily insulin dose, bolus, injections, prime insulin records, basal rate insulin records. For each result type and value, a date, time, time slot, device serial number, comment, and status may also be included.

The Summary Logbook report may include messages triggered by the Pattern Recognition analysis and glucose data for each of the defined time slots.

The Glucose Distribution report may include messages triggered by the Pattern Recognition analysis and a histogram of the glucose data. Each bar for each glucose range of the histogram may be stacked to represent the number of readings flagged as pre-meal, post-meal, fasting, bedtime, and not flagged.

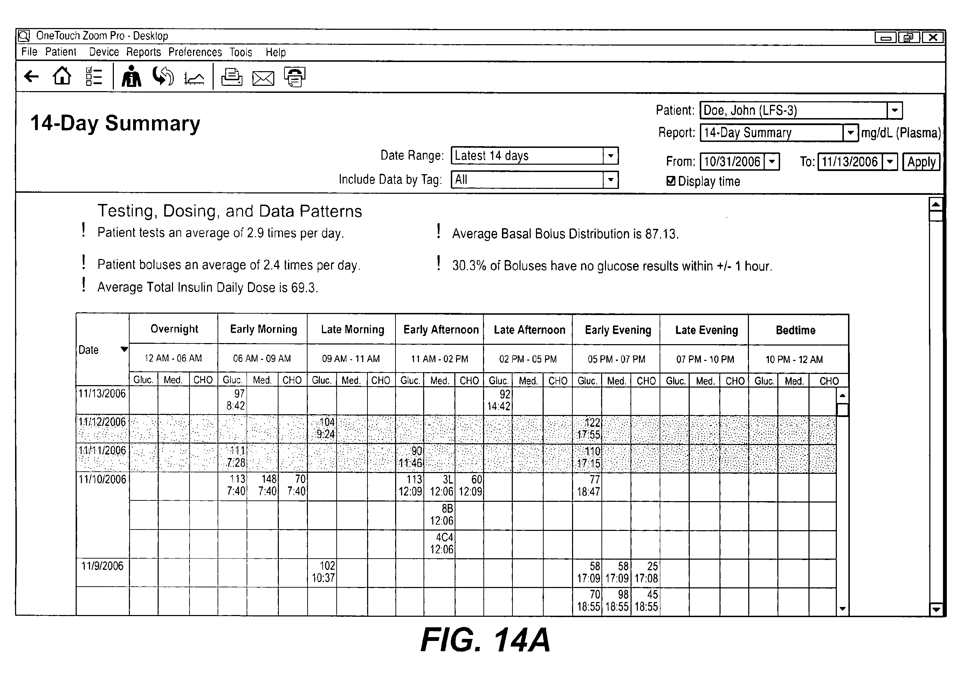

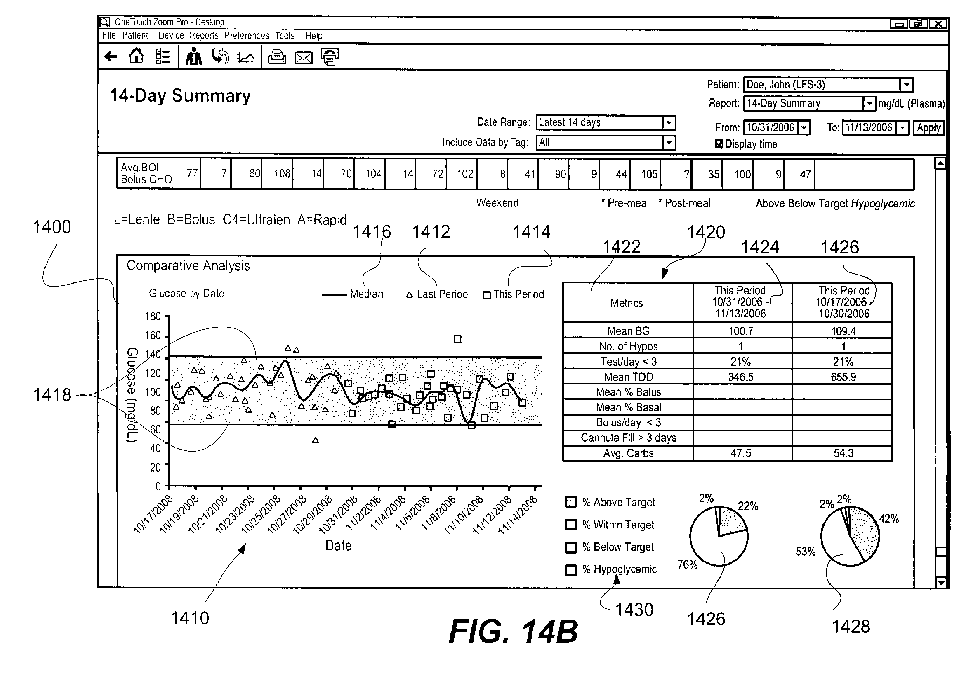

The 14-Day Summary report may include messages triggered by the Pattern Recognition analysis by a suitable microprocessor based device; a statistics table that analyzes glucose, insulin bolus, and carbohydrate data; a logbook portion that include glucose, insulin, and carbohydrate data for each of the defined time slots; a trend glucose concentration graph by date; and a pie chart, as illustrated in Figures 14A and 14B. The statistic table for a 14 day period may include a mean glucose concentration, a number of hypoglycemic results, a % (percent) of days with less than 3 glucose test per day, a mean total daily dose of insulin, a mean % (percent)basal dose, a mean % (percent)bolus dose, a % (percent) of days with less than 3 boluses per day, a % (percent) of days with greater than 3 days between cannula fills, and an average amount of carbohydrates. The logbook portion may include an array of cells, each cell including a glucose concentration for a particular time period, and optionally lifestyle data. The trend glucose concentration graph by date may include a plurality of glucose concentrations as a function of time, where each glucose concentration on the graph is selected by a user that causes a corresponding cell of the logbook to be highlighted. For example, a user can perform a single or double mouse click on a glucose concentration data point on the trend graph (see Figure 14B) that causes a corresponding cell of the logbook (see Figure 14A) to be highlighted.

The microprocessor may provide a useful comparative analysis 1400 (Figure 14B) to the user or HCP. In particular, the comparative analysis 1400 is provided by the microprocessor of a DMU on a suitable communication medium such as, for example, a paper print out, an electronic display, audio output or both audio and visual output of the analysis. The comparative analysis 1400 includes (as shown by way of example on the display screen of an HCP's computer) a trend graph 1410 of data relating to at least a physiologically related metric (e.g., blood glucose, ketone, insulin delivered or similar metrics relating to diabetes) for comparison between different reporting periods 1412 and 1414. In the trend graph of Figure 14B, the data selected for comparative analysis may be the median 1416 of the blood glucose data collected over the two periods 1412 and 1414 selected for comparison. The graph 1410 may be provided with a desired range indicator 1418 such as, for example, between 70 mg/dL and 140 mg/dL for the collected blood glucose data over the two reporting periods 1412 and 1414 as delineated by squares and triangles in the graph 1410.

The comparative analysis 1400 may also be provided with a table 1420 that provides a display of the data in an alphanumeric table. The selected metrics, a reporting period and another reporting period are provided in respective columns 1422, 1424, 1426 with data for each column disposed in respective rows. Metrics of interest are populated in the respective rows (e.g., mean glucose concentration, a number of hypoglycemic results, a % (percent) of days with less than 3 glucose test per day, a mean total daily dose of insulin, a mean % (percent)basal dose, a mean % (percent)bolus dose, a % (percent) of days with less than 3 boluses per day, a % (percent) of days with greater than 3 days between cannula fills, and an average amount of carbohydrates) between different reporting periods.

The comparative analysis 1400 may also be provided with pie-charts 1428 and 1430 for respective reporting periods. The pie-charts are also provided with a key indicator 1432 relating to percent of a selected metric (e.g., blood glucose) above a user defined target; percent of the selected metric within the user-defined target; percent of the selected metric below the user-defined target; and percent of the selected metric that would constitute a hypoglycemic state.

Referring back to Figure 10, in a step 2006 of Figure 10, a first type of medical device can be coupled to the data management unit. In an embodiment, glucose meter 10 may be the first type of medical device and personal computer 1026 may be the data management unit. The coupling may either be wireless or through physically attaching a cable between the devices. The data management unit may continuously or semi-continuously poll for a medical device to communicate with and then start the coupling process.

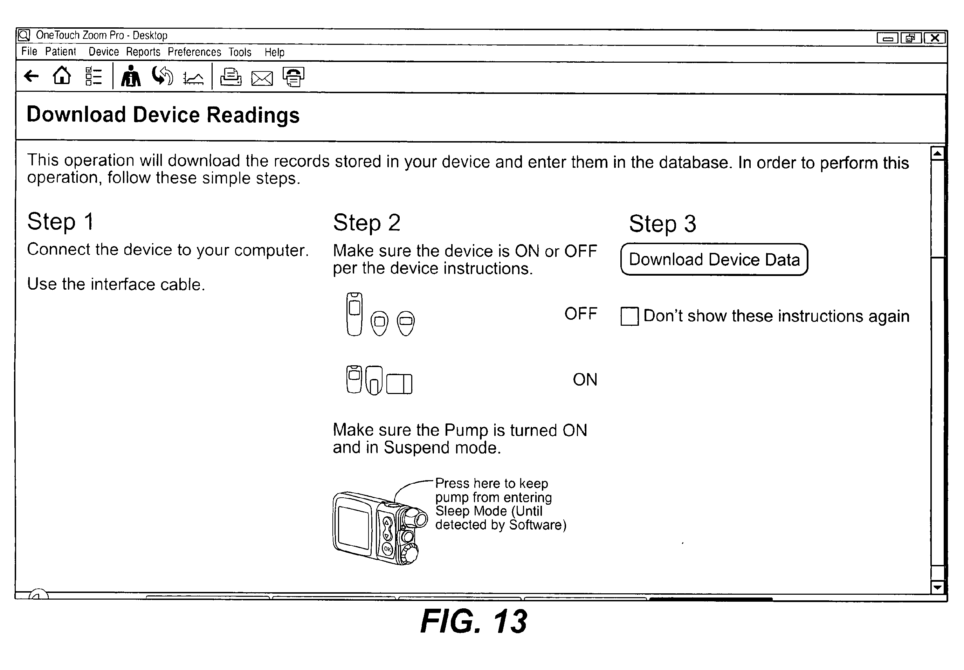

Once coupled, a user can provide an input into the user interface to trigger the output of a particular customized report, as illustrated in a step 2008. Figure 13 illustrates an example of a user interface where the user can perform a single mouse click on a user interface button "Download Device Data" to provide the trigger input. The data management unit can be configured to transfer data from the medical device, as shown in a step 2010, and then automatically recognize the medical device type (e.g., glucose meter or insulin pump) that has been coupled, as shown in a step 2012. Next, the user interface can output a customized report that was pre-selected for the recognized medical device, as shown in a step 2014. Note that in an embodiment the Summary by Day report can be annunciated for a glucose meter and that the Data List report can be annunciated for an insulin pump. The output report may be displayed on a screen of the data management unit, automatically printed by a printer, e-mailed, texted, and/or faxed.

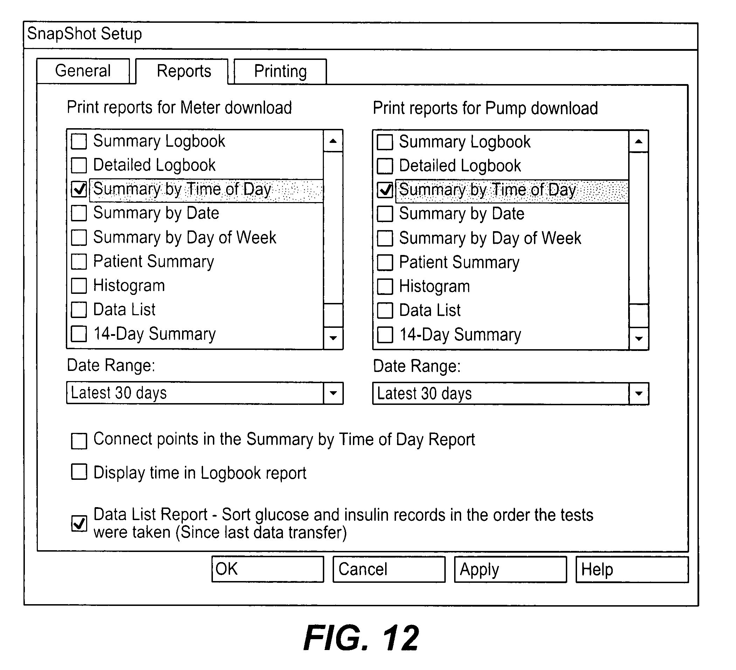

Alternatively, a user can pre-select a different set of customized reports associated with a particular type of medical devices that are automatically outputted without an input into a user interface, as illustrated in a step 2016. Figure 12 illustrates an exemplary screen-shot of a user interface that allows a user to pre-select one or more customized reports that correspond to a glucose meter and to an insulin pump. In step 2016, the customized reports are automatically generated once the medical device is coupled and automatically recognized. The coupling and transfer of data may also occur automatically once the medical device is recognized.

Diabetes management software that transfers data from medical devices to data management units can be used by HCP's to analyze their patient population as a group. HCP's can use the software to focus in on patients who are at high risk or with hard to control blood glucose. In addition, HCP's can use the software to organize a clinical study by screening for a particular group of patients who meet the desired demographics and/or data criteria for the study.

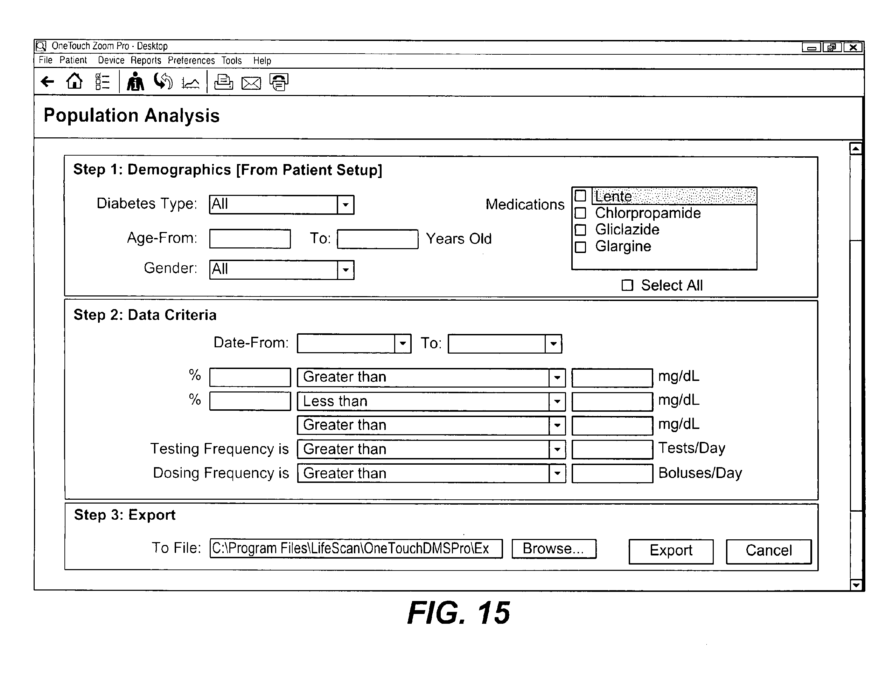

Figure 15 illustrates a screen-shot of a user interface for doing a population analysis. In an embodiment, demographics can include a diabetes type of the patient (e.g., Type 1 or 2), age, gender, and medication type (e.g., oral type of medication or insulin type of medication). In an embodiment, data criteria can include a % (percent) of glucose results greater than a high blood glucose threshold or less than a low blood glucose threshold, a mean glucose value higher than a high glucose threshold or less than a low glucose threshold, a testing frequency more than a high threshold or less than a low threshold, an insulin dosing frequency more than a high threshold or less than a low threshold. Note that the various thresholds can be configured by the HCP based on the type of clinical study being organized. The HCP can use any combination of demographics and data criteria described above to select a certain patient population to manage risk, improve outcomes, or to do clinical studies. In an embodiment, the population analysis method can include both insulin pump data as well as glucose meter data.

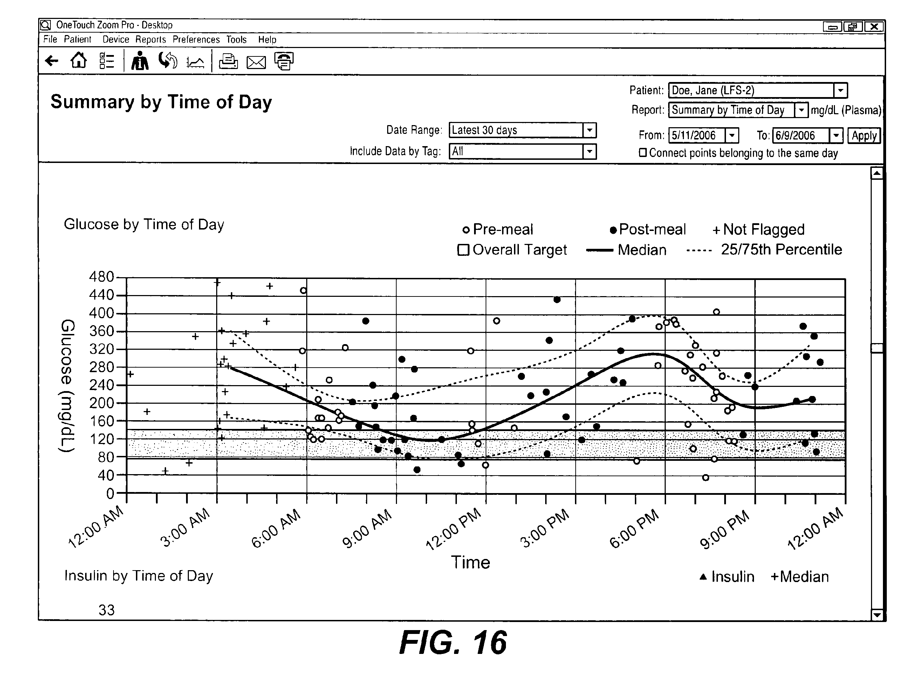

As mentioned above, trend or scatter graphs can be used in analyzing a person with diabetes disease state. However, when there are a large number of data points on one plot, applicants believe that software tools to filter the data based on events will provide a clearer picture of the data trend event by event. Figure 16 illustrates a glucose trend graph that includes pre-meal flags, post-meal flags, and not flagged glucose measurements. Because several types of flags are grouped together in Figure 16, this may, under certain circumstances, create confusion in the mind of the HCP. The "flags" can refers to patient generated events where the patient makes a note in a glucose meter that a certain glucose result was taken during fasting (right before breakfast for instance), bedtime or the patient notes a pre-meal or post-meal event. In an embodiment, the user may press a button on a glucose meter to save a particular type of flag in the memory of the meter, which will then be associated with the last glucose result. In addition, the user may have the option to not flag the glucose result, which creates a "Not Flagged" result.

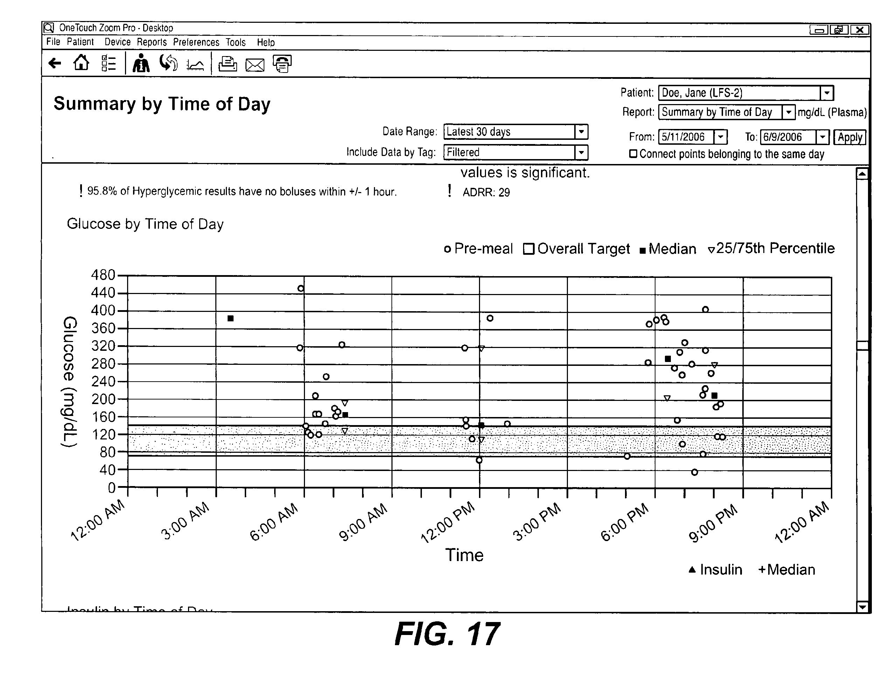

To provide an easier to interpret trend graph, a user can filter the data based on specific flags or no flags. Such trend graphs can be viewed by time of day, by date, by day of week, or in a histogram format. In an embodiment, the data in Figure 16 can be filtered for pre-meal flags, as is illustrated in Figure 17. After the filtering step, a user or HCP can clearly see that the user has high pre-meal results primarily around dinner and breakfast.

it is noted that the various methods described herein can be used to generate software codes using off-the-shelf software development tools such as, for example, Visual Studio 6.0, Windows 2000 Server, and SQL Server 2000. The methods, however, may be transformed into other software languages depending on the requirements and the availability of new software languages for coding the methods. Additionally, the various methods described, once transformed into suitable software codes, may be embodied in any computer-readable storage medium that, when executed by a suitable microprocessor or computer, are operable to carry out the steps described in these methods along with any other necessary steps.

While the invention has been described in terms of particular variations and illustrative figures, those of ordinary skill in the art will recognize that the invention is not limited to the variations or figures described. In addition, where methods and steps described above indicate certain events occurring in certain order, those of ordinary skill in the art will recognize that the ordering of certain steps may be modified and that such modifications are in accordance with the variations of the invention. Additionally, certain of the steps may be performed concurrently in a parallel process when possible, as well as performed sequentially as described above. Therefore, to the extent there are variations of the invention, which are within the spirit of the disclosure or equivalent to the inventions found in the claims, it is the intent that this patent will cover those variations as well.

## Claims

1. A method of transferring data from a medical device to a data management unit, the data management unit including a processor and configured to communicate with a plurality of medical device types, the method comprising:
generating with the microprocessor, different customized reports specific to different medical device types;
coupling the at least one type of the plurality of medical device types to the data management unit;
identifying with the microprocessor, that the at least one type of medical device types has been coupled to the data management unit;
providing an input into the user interface to trigger the output of customized report specific to the at least one type of medical device;
and
outputting the customized report.

2. The method of claim 1 further comprising:
outputting another customized report specific to another type of the plurality of medical device types automatically without user input once the another medical device type has been identified.

3. The method of claim 1, further comprising transferring automatically data from one or more of the plurality of medical device types to the data management unit.

4. The method of claim 3, in which the data comprises at least one of blood glucose values of a subject.

5. The method of Claim 1, in which the coupling comprises a wireless coupling between the medical device and the data management unit.

6. The method of Claim 1, in which the coupling comprises a cable connecting the medical device and the data management unit.

7. The method of claim 1, in which the providing comprises clicking once on a user interface switch.

8. The method of claim 6, in which the plurality of medical device types comprise a glucose meter, a continuous glucose monitor, an insulin pen, or an insulin pump.

9. The method of claim 1, in which the customized report comprises a logbook or a graph, the logbook including an array of cells, each cell including a glucose concentration for a particular time period, and lifestyle data, the graph including a plurality of glucose concentrations as a function of time, the method further comprising:
selecting a glucose concentration on the graph; and
highlighting a cell of the logbook that corresponds to the selected glucose concentration.

10. The method of claim 9, in which lifestyle data comprises information selected from the group consisting of exercise, diet, or medication and combinations thereof.

11. A diabetes management system comprising:
a data management unit; and
one or more medical device types,
in which the data management unit is configured to generate different customized reports specific to different medical device types based on a medical device type being coupled to the data management unit, and
in which the data management unit is configured to couple to the medical device so that the data management automatically identify the medical device type, transfer data from the identified medical device, and output the customized report specific to the identified medical device after an input on a user interface.

12. The diabetes management system of claim 11, in which the data management unit is configured to perform one of the following:
automatically poll for medical devices;
wirelessly couple to one or more medical device types; or
couple to one or more medical device types with a wire.

13. The diabetes management system of claim 11, in which the input comprises a single mouse click on the user interface.

14. The diabetes management system of claim 11, in which the medical device is selected from a group consisting of a glucose meter, a continuous glucose monitor, an insulin pen, and an insulin pump.

15. The diabetes management system of claim 11, in which the customized report comprises a logbook and a graph, the logbook including an array of cells, each cell including a glucose concentration for a particular time period, and optionally lifestyle data, the graph including a plurality of glucose concentrations as a function of time, in which each glucose concentration on the graph is selectable by a user so that a corresponding cell of the logbook is highlighted.

16. The diabetes management system of claim 15, in which lifestyle data is information selected from the group consisting of exercise, diet, or medication, and combinations thereof.

17. The diabetes management system of claim 11, wherein the data management unit comprises:
a communication medium coupled to the microprocessor, the communication medium providing comparative data of a mean blood glucose, number of hyperglycemic incidence, number of day with test less than a specified value, and mean total daily dose of insulin collected over different periods of reporting.

18. The unit of claim 17, in which the comparative data comprises a % (percent) of days with less than 3 glucose test per day, a mean % (percent) basal dose, a mean % (percent) bolus dose, a % (percent) of days with less than 3 boluses per day, a % (percent) of days with greater than 3 days between cannula fills, and an average amount of carbohydrates for each of the different reporting period.

19. The unit of claim 18, in which the communication medium comprises a display of the data in an alphanumeric table in which selected metrics, a reporting period and another reporting period are provided in respective columns with data for each column disposed in respective rows.

20. The unit of claim 17, in which the communication medium comprises a display of:
the data in the form of pie-charts for respective reporting periods and indicator keys related to percent of a selected metric above a user defined target, percent of the selected metric within a the user-defined target, percent of the selected metric below the user-defined target, and percent of the selected metric reflective of a hypoglycemic state; or
a logbook and a graph, the logbook including an array of cells, each cell including a glucose concentration for a particular time period and lifestyle data, the graph including a plurality of glucose concentrations as a function of time, in which each glucose concentration on the graph is selectable by a user so that a corresponding cell of the logbook is highlighted.
